# EUROPEAN PATENT APPLICATION

(11) **EP 4 455 160 A1**
(43) Date of publication of application: **30.10.2024**
(21) Application number: 22906648.5
(22) Date of filing: 15.12.2022
(51) Int. Cl.: C07K 14/755, A61K 48/00

(54) **PRODUCT AND METHOD FOR TREATING THROMBOCYTOPENIA**

(30) Priority: 15.12.2021 CN 202111534894
(71) Applicant: SHANGHAI SYNVIDA BIOTECHNOLOGY CO. LTD., Shanghai 201303 (CN)
(72) Inventor: XU, Yanyan, Shanghai 201203 (CN); LIU, Junling, Shanghai 201203 (CN); LIU, Yanjun, Shanghai 201203 (CN); WANG, Yanhua, Shanghai 201203 (CN)
(74) Representative: Delbarba, Andrea
(86) International application number: PCT/CN2022/139309
(87) International publication number: WO 2023/109903

(57) **Abstract**

Provided are a product and a method for treating thrombocytopenia. The product and the method can fundamentally solve the problem of thrombocytopenia from the perspective of an autoimmune mechanism.

## Description

### FIELD OF THE INVENTION

The present disclosure relates to the field of biology and pharmacy. More specifically, the present disclosure refers to a use of platelet surface antigen or a truncated fragment thereof for treating thrombocytopenia-related diseases.

### BACKGROUND

Immune thrombocytopenia (or idiopathic thrombocytopenia), also known as idiopathic thrombocytopenic purpura (ITP), is an autoimmune disease that causes bleeding due to peripheral thrombocytopenia, accounting for about 1/3 in the total number of bleeding diseases. Currently, about one-third of patients with immune thrombocytopenia (ITP) respond poorly to pharmacological treatment in clinical, eventually progressing to chronic ITP (cITP) with a high mortality rate.

The main pathogenesis of immune thrombocytopenia involves the proliferation of autoreactive B lymphocytes within the body, with secreted autoantibodies (PA Abs) specific to glycoproteins on the surface of platelet membranes. These PA Abs exhibit a specificity as high as 90% in ITP patients. The binding of these antibodies to platelets causes phagocytosis and clearance of platelets by the reticuloendothelial system in the spleen, thereby impairing their normal hemostatic functions. At the same time, PA Abs can bind to megakaryocytes, affecting platelet maturation and differentiation, which is the major cause of ITP. PA Abs-positive ITP patients have a higher risk of severe bleeding, poorer efficacy and prognosis than PA Abs-negative patients.

Current clinical treatment for ITP is mainly aimed at improving the symptoms of patients. Among them, first-line treatments include glucocorticoids, γ-globulin, immunoglobulin infusion, platelet transfusion and other methods. However, these treatments have limited efficacy and significant side effects (e.g., hormones), with a high proportion of relapsed and refractory cases. Additionally, blood product sources are extremely scarce, with a risk of infection. Second-line treatments include immunosuppressants, splenectomy, thrombopoietic agents and non-specific B lymphocyte depletion (e.g., CD20 antibodies). The application of immunosuppressants and CD20 antibodies can indiscriminately destroy the already dysregulated immune system, leading to secondary infections and other issues. Under the stimulation of thrombopoietic agents, platelets produced and continued to be used as antigens to further activate autoimmunity, resulting in their rapid clearance. This leads to issues of unstable efficacy and dual risks of thrombosis and hemorrhage. Moreover, these drugs are associated with severe adverse reactions such as bone marrow fibrosis and liver dysfunction. Therefore, it is urgent to find more effective treatment strategies in clinical practice.

### SUMMARY

The aim of the present disclosure is to provide a use of truncated platelet surface antigen for treating immune thrombocytopenia-related diseases.

The aim of the present disclosure is also to provide a chimeric autoantibody receptor (CAAR) prepared by using the truncated platelet surface antigen or a variant thereof and uses thereof.

In the first aspect, the present disclosure provides a use of the platelet surface antigen GpIbα, a truncated fragment, a variant, or an encoding polynucleotide thereof, for: (a) preparing a competitive antigen or a construct or composition comprising the antigen, the competitive antigen can bind to anti-platelet antibody, (b) preparing a construct or composition for targeting and eliminating reactive B lymphocytes, (c) preparing a construct or composition for increasing platelets, (d) preparing for a construct or composition for alleviating or treating immune thrombocytopenic purpura.

In one or more embodiments, the truncated fragment or the variant of GpIbα has the following characteristics: it has binding sites for in vivo platelet autoantibody; preferably, the truncated fragment or the variant of GpIbα is derived from the extracellular region of the GpIbα protein.

In one or more embodiments, the truncated fragment comprises a protein with the sequence of SEQ ID NO: 2, SEQ ID NO: 4, SEQ ID NO: 6, SEQ ID NO: 8, SEQ ID NO: 10, SEQ ID NO: 17, SEQ ID NO: 19 or SEQ ID NO: 21.

In one or more embodiments, the construct is Chimeric Auto-Antibody Receptor (CAAR).

In one or more embodiments, the anti-platelet antibody comprises B cell surface anti-platelet BCR and/or free anti-platelet antibody.

In another aspect, the present disclosure provides a truncated fragment of platelet surface antigen GpIbα, a variant, or an encoding polynucleotide thereof, wherein the truncated fragment or the variant has the following characteristics: it derived from the extracellular region of the GpIbα protein and it has binding sites for in vivo platelet autoantibody; preferably, the truncated fragment or the variant is a variant with a mutation in the binding region of von Willebrand factor (vWF) and decreased binding ability; more preferably, in the truncated fragment or the variant, residue 233 of the amino acid sequence as shown in SEQ ID NO: 2 is mutated from G to K (G233K); more preferably, the truncated fragment or the variant comprises one or more proteins with the following sequences: SEQ ID NO: 2, SEQ ID NO: 4, SEQ ID NO: 6, SEQ ID NO: 8, SEQ ID NO: 10, SEQ ID NO: 17, SEQ ID NO: 19 or SEQ ID NO: 21.

In one or more embodiments, the truncated fragment of the platelet surface antigen GpIbα or the variant binds anti-platelet antibody expressed by reactive B lymphocytes or free anti-platelet antibody secreted by plasma cells through platelet autoantigens, reducing killing effect of the antibody to platelets, increasing platelet counts.

In one or more embodiments, the binding is competitive binding.

In one or more embodiments, the truncated fragment of the platelet surface antigen GpIbα or the variant will not serve as an antigen for reactivating B lymphocytes to produce more autoimmune antibodies.

In one or more embodiments, the truncated fragment of the platelet surface antigen GpIbα or the variant is used as a competitive antigen specifically for plasma cells secreting free autoantibodies against platelet antigens or B lymphocytes expressing anti-platelet BCR, but B lymphocytes expressing normal BCR are not affected.

In one or more embodiments, the elimination comprises but are not limited to: decrease, inhibition, reduction, dissolution or retardation; preferably a statistically significant decrease, inhibition, reduction, dissolution or retardation, such as a reduction of reactive B lymphocytes and (or) free anti-platelet antibody to below 90%, 80%, 60%, 50%, 30%, 20%, 10%, 5% or 2% of the original content.

In one or more embodiments, the truncated fragment is encoded by a polynucleotide with the following nucleotide sequence: SEQ ID NO: 1, SEQ ID NO: 3, SEQ ID NO: 5, SEQ ID NO: 7 , SEQ ID NO: 9, SEQ ID NO: 16, SEQ ID NO: 18 or SEQ ID NO: 20; or a degenerate sequence of SEQ ID NO: 1, SEQ ID NO: 3, SEQ ID NO: 5, SEQ ID NO: 7 , SEQ ID NO: 9, SEQ ID NO: 16, SEQ ID NO: 18 or SEQ ID NO: 20.

In one or more embodiments, the variant comprises: (1) a protein formed by one or more (such as 1-20; preferably 1-15; more preferably 1-10, such as 5, 3, 1) amino acid residue substitution, deletion or addition in the amino acid sequence of the truncated fragment, also with the function of truncated fragment; (2) a protein with more than 80% (preferably more than 85%; more preferably more than 90%; more preferably more than 95%, for example 98%, 99%) homology to the amino acid sequence of the truncated fragment, also with the function of the truncated fragment; (3) a fused protein comprising the truncated fragment.

In one or more embodiments, the amino acid sequence of the variant is as shown in SEQ ID NO: 17, SEQ ID NO: 19 or SEQ ID NO:21.

In one or more embodiments, the reduction of the binding ability is a statistically significant reduction, such as a reduction of 5%, 10%, 15%, 20%, 30%, 50%, 70%, 80%, 90%, 95% or more.

In another aspect, the present disclosure provides a chimeric autoantibody receptor, comprising: the truncated fragment of platelet surface antigen GpIbα or the variant as an extracellular binding region, a transmembrane domain and an intracellular signaling domain; preferably, the intracellular signaling domain comprises: co-stimulatory molecules and/or signaling transduction domains.

In one or more embodiments, the transmembrane domain comprises CD8 or CD28 hinge region and transmembrane region; preferably, the transmembrane domain comprises CD8 hinge region and transmembrane region; more preferably, the hinge region has an amino acid sequence of residues 291(T) to 335(D) as shown in SEQ ID NO: 11, the transmembrane region has an amino acid sequence of residues 336(I) to 359(C) as shown in SEQ ID NO: 11.

In one or more embodiments, the intracellular signaling domain comprises an intracellular signaling region selected from 4-1BB, CD3ζ, FcεRIγ, CD27, CD28, CD134, ICOS or GITR; preferably, the intracellular signaling region comprises 4-1BB and CD3ζ; more preferably, the 4-1BB has an amino acid sequence of residues 360(K) to 401(L) as shown in SEQ ID NO:11, the CD3ζ has an amino acid sequence of residues 402(R) to 513(R) as shown in SEQ ID NO: 11.

In one or more embodiments, the chimeric autoantibody receptor comprises the following sequentially connected structures: the truncated fragment of platelet surface antigen GpIbα or the variant, CD8 or CD28 hinge region and transmembrane region, 4-1BB and CD3ζ; preferably, the chimeric autoantibody receptor has any one of amino acid sequences as shown in SEQ ID NO: 11 to SEQ ID NO: 15.

In one or more embodiments, in the chimeric autoantibody receptor, the truncated fragment of platelet surface antigen GpIbα preferably has an amino acid sequence as shown in SEQ ID NO: 11.

In one or more embodiments, in the chimeric autoantibody receptor, the truncated fragment of platelet surface antigen GpIbα preferably is a variant, the variant is a variant with a mutation in the binding region of von Willebrand factor (vWF) and decreased binding ability; more preferably, in the variant, residue 233 of the amino acid sequence as shown in SEQ ID NO: 2 is mutated from G to K (G233K).

In one or more embodiments, the chimeric autoantibody receptor will not serve as an antigen for reactivating B lymphocytes to produce more autoimmune antibodies.

In one or more embodiments, the chimeric autoantibody receptor specifically targets and kills for plasma cells secreting free autoantibodies against platelet antigens or B lymphocytes expressing anti-platelet BCR, but B lymphocytes expressing normal BCR are not affected

In one or more embodiments, the chimeric autoantibody receptor has limited or no toxicity to healthy cells.

In another aspect, the present disclosure provides an expression construct, comprising: (i) a polynucleotide encoding the chimeric autoantibody receptor; or (ii) a polynucleotide encoding the truncated fragment of platelet surface antigen GpIbα or the variant.

In one or more embodiments, the expression construct comprises an expression vector.

In one or more embodiments, the vector is an RNA vector.

In one or more embodiments, the expression vector comprises: a viral vector or a non-viral vector.

In one or more embodiments, the expression vector is a viral vector.

In one or more embodiments, the viral vector comprises (but is not limited to): a lentiviral vector, an adenoviral vector or an adeno-associated viral vector.

In one or more embodiments, the virus comprises (but is not limited to): lentivirus, adenovirus or adeno-associated virus.

In one or more embodiments, the expression construct comprises a promoter operably linked to the polynucleotide encoding the chimeric autoantibody receptor.

In one or more embodiments, the expression construct comprises a promoter operably linked to the polynucleotide encoding the truncated fragment of platelet surface antigen GpIbα or the variant.

In one or more embodiments, the promoter is a constitutive expression promoter.

In one or more embodiments, the promoter is an inducible expression promoter, comprising a spatiotemporal inducible promoter, a tissue inducible promoter, and so on.

In another aspect, the present disclosure provides a virus, the virus (comprising but not limited to lentivirus) comprises the expression vector.

In another aspect, the present disclosure provides an immune effector cell, wherein it is introduced with: the polynucleotide of the chimeric autoantibody receptor or the expression construct; or expressing the chimeric autoantibody receptor.

In one or more embodiments, the immune effector cell comprises the cells selected from the following group or a combination thereof: T cell, natural killer cell (NK), NKT cell or cytokine-induced killer cell (CIK); more preferably, the immune effector cell is T cell; more preferably, the T cell comprises: helper T cell, cytotoxic T cell, memory T cell, regulatory T cells, γδ T cells, T memory stem cell or T cell derived from pluripotent stem cell.

In one or more embodiments, the T cell is an immune effector T cell with cytotoxicity.

In one or more embodiments, the chimeric autoantibody receptor expressed by the immune effector cell has a high affinity for the B lymphocyte receptor (BCR) based on the autoantibody located on B lymphocytes.

In one or more embodiments, the immune effector cell further expresses cytokines with immunoregulatory activity or able to enhance immune effector cell functions; preferably, the cytokines comprise (but are not limited to): IL-12 (stimulates the proliferation of immune cells), IL-15 (activates immune cells), IL-21 (improves the activity of immune cells), IL-2 (stimulates and maintains T cell differentiation and proliferation), IL-4 (regulates the activity of immune cells), IL-7 (regulates the maturation, activation, proliferation and immune regulation of immune cells), IL-9 (regulates the growth or activity of immune cells), IL-17 (promotes the activation of immune cells), IL-18 (promotes the proliferation of immune cells), IL-23 (promotes the proliferation of immune cells).

In another aspect, the present disclosure provides a use of the immune effector cell for: (a) preparing a composition for neutralizing anti-platelet antibody or free antibody, (b) preparing a composition for targeting and eliminating reactive B lymphocytes, (c) preparing a composition for increasing platelets, (d) preparing a composition for alleviating or treating immune thrombocytopenic purpura.

In another aspect, the present disclosure provides a pharmaceutical composition or drug kit, comprising the immune effector cell, the pharmaceutical composition or drug kit is used for: (a) neutralizing anti-platelet antibody or free antibody, (b) targeting and eliminating reactive B lymphocytes, (c) increasing platelets, (d) alleviating or treating immune thrombocytopenic purpura.

In one or more embodiments, the pharmaceutical composition also comprises a pharmaceutically acceptable carrier or excipient.

In one or more embodiments, the drug kit comprises a container/package, and the immune effector cell or the pharmaceutical composition comprising the immune effector cell placed in the container/package.

In another aspect, the present disclosure provides a method for (a) neutralizing anti-platelet antibody or free antibody, (b) targeting and eliminating reactive B lymphocytes, (c) increasing platelets, (d) alleviating or treating immune thrombocytopenic purpura, wherein the method comprises: administering an effective amount of the immune effector cell or the pharmaceutical composition to a subject.

In one or more embodiments, the immune thrombocytopenic purpura (ITP) is a disease caused by various primary or secondary causes.

In one or more embodiments, the subject with thrombocytopenia (reduced platelet count) is a subject whose peripheral blood platelet count is statistically lower than the normal peripheral blood platelet count in a healthy subject, such as a reduction of 1%, 2%, 3%, 5%, 8%, 10%, 12%, 15%, 20%, 25%, 30%, or a greater reduction.

In one or more embodiments, the subject with thrombocytopenia (reduced platelet count) is a relapsed refractory subject.

In one or more embodiments, the relapsed refractory subject has received at least one ITP treatment.

Other aspects of the present disclosure will be apparent to those skilled in the art based on the disclosure herein.

### BRIEF DESCRIPTION OF DRAWINGS

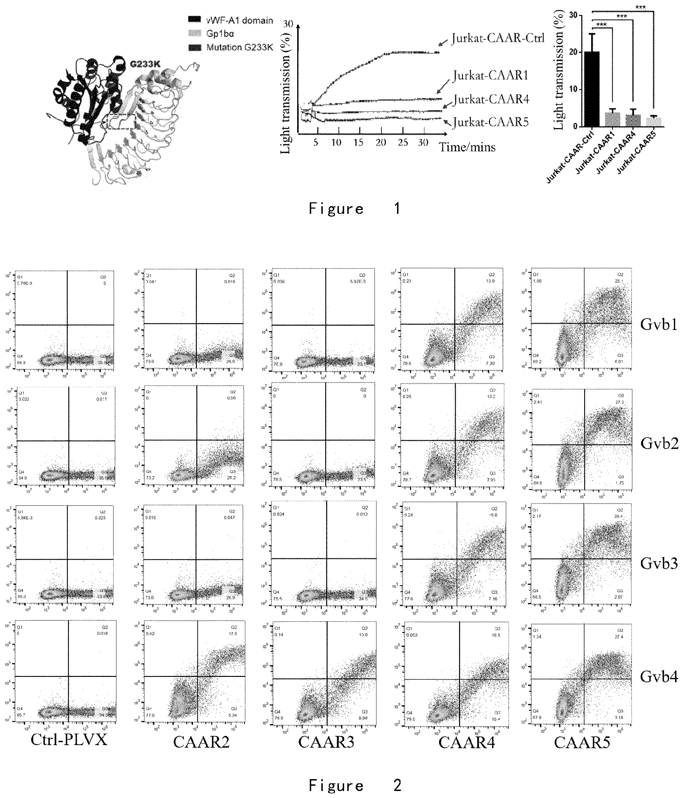

**Figure 1****. The effect of G233K point mutation on the aggregation of CAAR-T cells and vWF detected by aggregation reaction.** Under the action of ristocetin, the aggregation of Jurkat cells stably expressing CAAR1, CAAR4 and CAAR5 mutants with human vWF was measured by cell aggregation assay. The results showed that the aggregation of Jurkat cells expressing CAAR1, CAAR4 and CAAR5 mutants with vWF was significantly lower than that of Jurkat cells without point mutations (decreased light transmission), indicating that G233K point mutation leads to a significantly decreased ability of Jurkat-CAAR-T cells bound to human vWF.
**Figure 2****. Flow cytometry for screening the binding ability of GpIbα-CAAR-T cells to anti-GpIbα positive hybridoma cells.** The results showed that different CAAR-T cells selectively recognized and bound to anti-GpIbα-positive hybridoma cells (Gvb1-Gvb4). Compared with CAAR2-T cells and CAAR3-T cells, CAAR4-T cells and CAAR5-T cells have a stronger binding ability to Gvb1-Gvb4 positive hybridoma cells. Besides, the G233K point mutation does not affect the specific binding of CAAR-T cells to anti-GpIbα positive hybridoma cells.

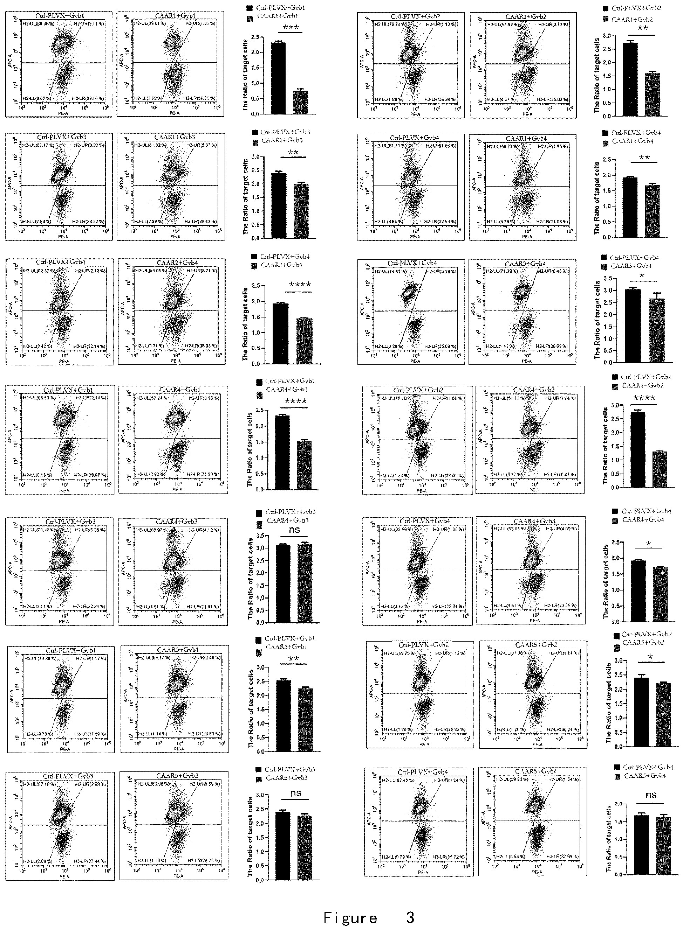

**Figure 3****. Flow cytometry for detecting the competitive cytotoxicity of CAAR-T cells to anti-GpIbα positive hybridoma cells.** The results indicated that GpIbα-CAAR-T cells could selectively recognize and competitively kill positive hybridoma cells with different antigen epitopes. Compared with CAAR2-T cells to CAAR5-T cells, CAAR1-T cells showed a strong competitive cytotoxicity against four positive hybridoma cells Gvb1 to Gvb4.

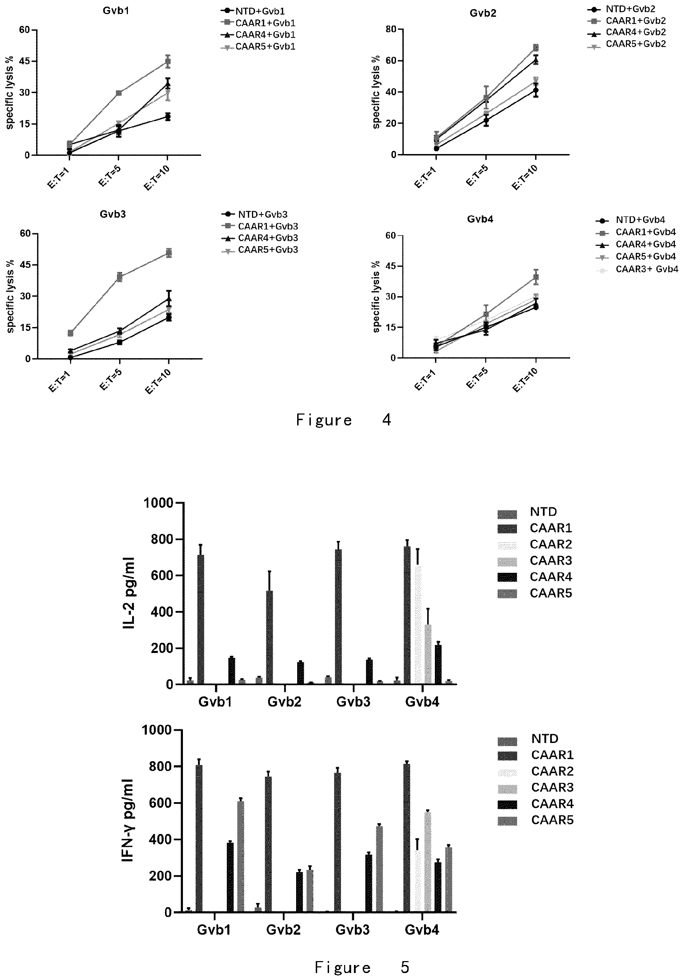

**Figure 4****. LDH release assay for measuring lysing effects of CAAR-T cells to anti-GpIbα positive hybridoma cells.** The results showed that CAAR1-T cells exhibit cytotoxicity against four types of positive hybridoma cells (Gvb1-Gvb4) at the same effect-to-target ratio, with the strongest effect of lysing cells, indicating that CAAR1-T cells possess a stronger cytolytic toxicity towards target cells compared to other CAAR-T cells.
**Figure 5****. ELISA assay for detecting anti-GpIbα positive hybridoma cytokines (IL-2 and IFN-γ) released by CAAR-T cells.** The results indicated that CAAR1-T cells exhibit cytotoxicity against four types of positive hybridoma cells (Gvb1-Gvb4), with the highest level of released IL-2 and IFN-γ, suggesting that CAAR1-T cells have stronger cytotoxicity against target cells compared to other CAAR-T cells.

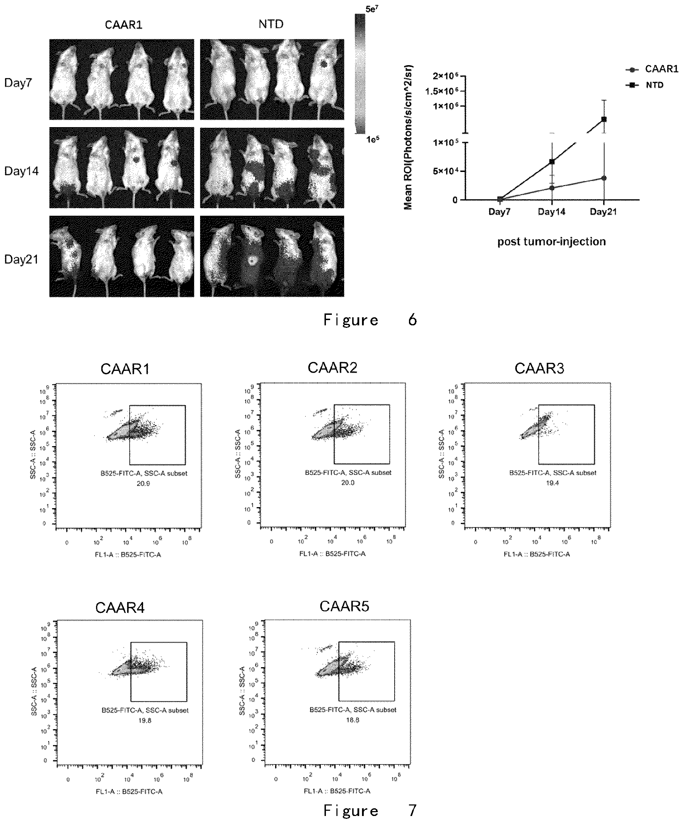

**Figure 6****. Luciferase fluorescence expression system for visualized observing the effect of CAAR-T on killing target cell Gvb1 in vivo.** The Gvb1 cell line was stably transduced with a GFP fluorescent reporter gene to enable real-time monitoring of Gvb1 cell growth and metastasis. Gvbl-GFP cells were injected into the tail vein of NCG mice, followed by the injection of either CAAR-T cells or control T cells. The survival status of mice was observed daily. Fluorescent images were photographed on days 7, 14, and 21 to monitor in-vivo Gvbl-GFP fluorescence of mice. As shown by the images and the statistic results, at the 14th day, the control group (NTD) mice showed significant Gvb1-GFP expansion and metastasis, while the CAAR-T cell-treated group exhibited significantly lower Gvbl-GFP cell expansion and metastasis than the control group. At the 21st day, the control group (NTD) mice displayed a significant degree of Gvbl-GFP expansion and metastasis, whereas the CAAR-T cell-treated group had significantly lower Gvb1-GFP cell expansion and metastasis than the control group, demonstrating a clear cytotoxic effect of CAAR-T cells on Gvb1 cells.
**Figure 7****. Construction of different truncated GpIbα-CAAR-T cells.** A sufficient number of T cells were obtained from the peripheral blood of volunteer subjects. The T cells were washed and activated in a sterile environment, and cultured with essential cytokines necessary for survival and proliferation. CD3 antibodies and CD28 antibodies were used for T cell activation in vitro. Activated T cells were infected with lentiviral vectors containing different truncated GpIbα-CAAR gene fragments and cultured for a period of time, flow cytometry results indicated that lentiviruses with different truncated fragments could infect a certain proportion of human T cells, thus constructing GpIbα-CAAR-T cells containing different truncated fragments.

### DETAILED DESCRIPTION

After extensive and in-depth research, the inventors have unexpectedly discovered for the first time that in patients with immune thrombocytopenia (immune thrombocytopenic purpura), extracellular protein fragments derived from the platelet surface antigen GpIbα, along with several truncated fragments and modified antigen fragments, can act as competitive antigens. These competitive antigens bind competitively with autoimmune antibodies (anti-platelet antibodies) expressed by reactive B lymphocytes and free antibodies secreted by plasma cells in vivo. Besides, these competitive antigens do not bind with natural ligands in vivo, thereby not affecting hemostasis and coagulation. Moreover, using these competitive antigens, the inventors further developed chimeric CAAR-T cells and IgG1 Fc fused proteins, which specifically target and kill plasma cells that secrete free autoantibodies against platelet antigens or B lymphocytes expressing anti-platelet BCRs, without affecting B lymphocytes expressing normal BCRs. Consequently, these cells and proteins reduce the cytotoxicity of platelets by autoimmune antibodies, thereby increasing platelet count and fundamentally addressing the issue of thrombocytopenia from the perspective of autoimmunity. Additionally, these competitive antigens and their modified chimeric CAAR-T cells or Fc fused proteins do not act as antigens to re-activate B lymphocytes, thus preventing the production of more autoimmune antibodies. On this basis, the present disclosure has been accomplished.

### Terms

Unless defined otherwise, all technical and scientific terms used herein have the meaning commonly understood by one of ordinary skill in the art to which this present disclosure belongs.

In the present disclosure, when referring to a measurable value such as amount, duration, etc., "about" as used herein is intended to cover ±20%, ±10%, ±5%, 2% or ±1% of the stated value, and ±0.5% or 0.1% deviation in some cases.

As used herein, "limited toxicity" means that the truncated fragments (peptides), polynucleotides, chimeric autoantibody receptors or cells of the present disclosure exhibit in vitro or in vivo effects on healthy cells, non-disease cells, non-target cells or populations of these cells with lacked substantially negative biological effects or physiological symptoms.

As used herein, "autoantibody" refers to an antibody produced by a B lymphocyte specific for a self-antigen.

As used herein, an "autoimmune disease" is defined as a disorder or condition resulting from an autoimmune response mediated by antibodies against self-antigens. Autoimmune diseases result in inappropriate and/or excessive production of autoantibodies against self-antigens or autoantibodies of self-antigens.

As used herein, "chimeric autoantibody receptor" or "CAAR" refers to an engineered receptor expressed on T cells or any other effector cell capable of mediating cell cytotoxicity. CAAR comprises antigens or fragments thereof specific for pathogenic autoantibodies. CAAR also comprises a transmembrane domain, an intracellular domain and a signaling domain.

As used herein, "effective amount" or "therapeutically effective amount" can be used interchangeably and refers to the amount of a compound, formulation, material or composition effective to achieve a particular biological result as described herein.

As used herein, "operably linked" or "linked operably" refers to a functional linkage between a regulatory sequence and a heterologous nucleic acid sequence, resulting in the expression of the latter. For example, a first nucleic acid sequence is operably linked to a second nucleic acid sequence when the first nucleic acid sequence is in a functional relationship with the second nucleic acid sequence. For example, a promoter is operably linked to a coding sequence if the promoter affects the transcription or expression of the coding sequence. Generally, operably linked DNA sequences are contiguous and, when necessary, two protein coding regions can be linked into the same reading frame.

As used herein, a "subject" comprises a living organism capable of eliciting an immune response, such as a mammal, comprising a human.

As used herein, "transmembrane domain" refers to the portion or region of a molecule that spans a bimolecular lipid membrane.

As used herein, "sample to be tested" or "sample to be evaluated" refers to a sample to be tested. In the present disclosure, the sample to be tested can be: plasma (such as from peripheral blood), isolated or processed samples comprising platelets, etc.

As used herein, the term "diagnosis" refers to a method used by those skilled in the art to assess and/or determine the likelihood of an individual suffering from a particular disease, disorder or symptom. The term "diagnosis" also comprises detecting a predisposition to developing a disease, disorder or symptom, determining the therapeutic efficacy of a drug treatment, or predicting a response to a drug treatment. The diagnostic methods of the present disclosure can be performed alone or concurrently with other diagnostic and/or staging methods for analyzing a disease, disorder or symptom.

### Truncated fragment of GpIbα

In most patients with immune thrombocytopenia (immune thrombocytopenic purpura), one or several autoantibodies against platelet membrane epitopes can be detected, wherein the membrane protein GPIbα is one of the main epitopes produced by autoantibodies. The present disclosure provides a series of truncated fragments of the extracellular region of GpIbα. They compete with platelet autoantigens in vivo for binding to autoimmune antibodies expressed by reactive B lymphocytes (anti-platelet antibodies) and free antibodies secreted by plasma cells. Moreover, the competitive antigen does not bind to the natural ligand in vivo and has no effect on blood coagulation. The truncated fragment of GpIbα has the following characteristics: it derived from the extracellular region of the GpIbα protein and it has binding sites for in vivo platelet autoantibody; preferably, the truncated fragment comprises a protein with the amino acid sequence of SEQ ID NO: 2, SEQ ID NO: 4, SEQ ID NO: 6, SEQ ID NO: 8, SEQ ID NO: 10, SEQ ID NO: 17, SEQ ID NO: 19 or SEQ ID NO: 21.

The present disclosure also comprises active fragments, derivatives and analogues of the truncated fragment. As used herein, the terms "active fragment", "derivative", and "analogue" refer to polypeptides that maintain essentially the same biological function or activity as the truncated fragment.

The protein fragments, derivatives or analogues of the present disclosure may be (i) proteins having one or more conservative or non-conservative amino acid residues (preferably conservative amino acid residues) substituted, and such substituted amino acid residues may or may not be encoded by the genetic code, or (ii) a protein having a substituent group in one or more amino acid residues, or (iii) a protein formed by an additional amino acid sequences fused to the protein sequence (such as leader sequence or secretory sequence or sequence used to purify the protein or proprotein sequence, or fused protein). According to the teaching herein, these fragments, derivatives, and analogues belong to the common knowledge to those skilled in the art.

In the present disclosure, the "truncated fragment of GpIbα" further includes (but is not limited to): deletion, insertion and/or substitution of one or more (usually 1-50, preferably 1-30, more preferably 1-20, most preferably 1-10, even more preferably1-8, 1-5) amino acids, and addition or deletion of one or several (usually within 20, preferably within 10, more preferably within 5) amino acids at the C-terminal and/or N-terminal. For example, in the art, substitution with amino acids of approaching or similar property generally does not alter the function of a protein. For another example, adding one or more amino acids to the C- terminal and/or N- terminal usually does not change the function of the protein.

In a preferable embodiment, in the amino acid sequence of the truncated fragment of GpIbα or active fragments, derivatives and analogs according to the present disclosure, there are preferably mutations at individual sites, so that the binding region that binds to vWF is mutated, also with a decreased binding ability; more preferably, the variant has a G233K mutation.

The polypeptide sequence described herein is preferably a polypeptide with an amino acid sequence as shown in SEQ ID NO: 2, SEQ ID NO: 4, SEQ ID NO: 6, SEQ ID NO: 8 or SEQ ID NO: 10. More preferably, there is also a G233K mutation. The polypeptide comprising the G233K mutation also comprises the sequence shown in SEQ ID NO: 17, SEQ ID NO: 19 or SEQ ID NO: 21.

In the present disclosure, the amino terminus or carboxyl terminus of the truncated fragment of GpIbα may also comprise one or more polypeptide fragments as protein tags. Any suitable tag may be used in the present disclosure. For example, the tags may be FLAG, HA, HA1, c-Myc, Poly-His, Poly-Arg, Strep-TagII, AU1, EE, T7, 4A6, ε, B, gE and Ty1. There tags may be used to purify proteins. In order to secrete and express the translated protein (such as secreted out of the cell), a signal peptide sequence, such as pelB signal peptide, can also be added to the amino acid terminal of the truncated fragment of GpIbα. The signal peptide can be cleaved during the secretion of the polypeptide from the cell.

The present disclosure also provides a polynucleotide sequence encoding the truncated fragment of GpIbα or a conservative variant protein thereof in the present disclosure. The polynucleotide may be in DNA form or RNA form. DNAs include cDNAs, genomic DNAs, or synthetic DNAs. DNAs can be single stranded DNAs or double stranded DNAs. DNAs can be coding or noncoding strand DNAs. The polynucleotide encoding the mature protein of the truncated fragment comprises: the coding sequence encoding only the mature protein; the coding sequence of the mature protein and various additional coding sequences; the coding sequence of the mature protein (and optional additional coding sequences) and non-coding sequence. The coding region sequence encoding the mature polypeptide may be the same as the coding region sequence of the polynucleotides provided in the examples or be a degenerate variant. As used herein, "degenerate variant" in the present disclosure refers to a nucleic acid sequence that encodes the truncated fragment of the present disclosure, but differs from the coding region sequence of the polynucleotides provided in the examples.

The present disclosure also relates to variants of the above-mentioned polynucleotides, wherein the variants encode proteins, or fragments, analogues and derivatives of the proteins, with the same amino acid sequences as the present disclosure. The variants of the polynucleotide may be naturally occurring allelic variants or non-naturally occurring variants. These nucleotide variants include degenerate variants, substitution variants, deletion variants, and insertion variants. As known in the art, an allelic variant is an alternative form of a polynucleotide, which may be the substitution, deletion, or insertion of one or more nucleotides, but does not substantially alter the function of the protein it encodes.

The nucleotide full-length sequence of the truncated fragment of GpIbα or fragments thereof in the present disclosure can usually be obtained by PCR amplification method, recombination method or artificial synthesis method. For the PCR amplification method, primers can be designed according to the relevant nucleotide sequences disclosed in the present disclosure, and the related sequences can be amplified. Related sequences can also be synthesized by artificial synthesis. This DNA sequence can then be introduced into various existing DNA molecules (or for example vectors) and cells known in the art. In addition, mutations can also be introduced into the protein sequence of the present disclosure through chemical synthesis.

The present disclosure also relates to vectors containing the polynucleotides of the present disclosure, as well as host cells produced by genetic engineering methods using the vectors or coding sequences of the truncated fragment of GpIbα of the present disclosure, and methods for producing the proteins of the present disclosure through recombinant technology.

Through conventional recombinant DNA technology, the polynucleotide sequences of the present disclosure can be used for expressing or producing truncated fragments of GpIbα. Generally speaking, steps are as follows: (1) transforming or transducing an appropriate host cell with a polynucleotide encoding the truncated fragment of GpIbα (or variants), or with a recombinant expression vector containing the polynucleotide; (2) culturing the host cells in a suitable medium; (3) isolating and purifying proteins from the culture medium or cells.

The present disclosure also relates to nucleic acid constructs comprising the polynucleotide sequences described herein, and one or more regulatory sequences operably linked to these sequences. The polynucleotides described herein can be manipulated in a variety of ways to guarantee expression of said polypeptide or protein. The nucleic acid construct can be manipulated according to the difference or requirements of the expression vector prior to insertion of the nucleic acid construct into the vector. Techniques that utilize recombinant DNA methods to alter polynucleotide sequences are known in the art.

In certain embodiments, the nucleic acid construct is a vector. The polynucleotide sequence of the truncated fragment of GpIbα can be inserted into the recombinant expression vector. The term "recombinant expression vector" refers to bacterial plasmids, phages, yeast plasmids, plant cell viruses, mammalian cell viruses or other vectors. In short, any plasmid and vector can be used as long as it can replicate and be stable in the hosts. An important characteristic of an expression vector is that it usually contains an origin of replication, a promoter, a marker gene, and translation control elements.

Those skilled in the art are well known of methods for constructing expression vectors comprising DNA sequences encoded by the truncated fragment of GpIbα and suitable transcription/translation control signals. These include in vitro recombinant DNA techniques, DNA synthesis techniques, in vivo recombination techniques, etc. Said DNA sequence can be effectively ligated to an appropriate promoter in an expression vector to direct mRNA synthesis. The expression vector also includes a ribosome binding site for translation initiation and a transcription terminator. In addition, the expression vector preferably comprises one or more selectable marker genes to provide a phenotypic trait for selection of transformed host cells.

Vectors comprising the appropriate DNA sequences described above, together with appropriate promoter or control sequences, can be used to transform appropriate host cells so that they can express the protein. Host cells can be prokaryotic cells, such as bacterial cells; or lower eukaryotic cells, such as yeast cells; or higher eukaryotic cells, such as plant cells, fungal cells, insect cells or mammalian cells. Representative examples comprise: prokaryotic cells such as *Escherichia coli, Bacillus subtilis, Streptomyces, Agrobacterium*; fungal cells such as yeast cells (such as *Pichia pastoris, Saccharomyces cerevisiae*) and so on. Transformation of host cells with recombinant DNA can be performed using conventional techniques known to those skilled in the art. The obtained transformants can be cultured to express the truncate fragments of the present disclosure.

### Chimeric AutoAntibody Receptor (CAAR)

The truncated fragment of GPIbα described in the present disclosure can be used for preparing a chimera with target cell-dependent cytotoxicity of platelet antibody. This chimera can be applied but not limited to the construction of CAAR-T cell immunotherapy drugs, exerting its effect on targeting and killing reactive B lymphocytes and reducing autoantibody titers. This can meet the clinical treatment needs for targeted elimination of PA Abs-reactive B lymphocytes.

Therefore, the present disclosure provides a CAAR expressed on the surface of immune effector cells, the CAAR comprises sequentially connected structures: an extracellular binding region, a transmembrane region and an intracellular signal region. These several structural domains are combined together. The CAAR is expressed in immune effector cells, making immune effector cells exert highly specific cytotoxicity on reactive B lymphocytes (B lymphocytes that cause autoimmune diseases). The extracellular binding region comprises a truncated fragment of the present disclosure or a variant thereof.

The extracellular binding region, the transmembrane region and the intracellular signal region may include a linker or not include a linker.

Examples of hinge and/or transmembrane domains comprise, but are not limited to: hinge and/or transmembrane domains of the α, β or ζ chains of T cell receptors, CD28, CD3ε, CD45, CD4, CD5, CD8, CD9, CD16, CD22, CD33, CD37, CD64, CD80, CD86, CD134, CD137, CD154, KIR, OX40, CD2, CD27, LFA-1(CD11a, CD18), ICOS(CD278), 4-1BB(CD137), GITR, CD40, BAFFR, HVEM(LIGHTR), SLAMF7, NKp80(KLRF1), CD160, CD19, IL2Rβ, IL2Ry, IL7Rα, ITGA1, VLA1, CD49a, ITGA4, IA4, CD49D, ITGA6, VLA-6, CD49f, ITGAD, CD11d , ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1(CD226), SLAMF4(CD244, 2B4), CD84, CD96, CEACAM1, CRTAM, Ly9(CD229), CD160(BY55), PSGL1, CD100(SEMA4D), SLAMF6(NTB-A, Ly108), SLAM(SLAMF1, CD150, IPO-3), BLAME(SLAMF8), SELPLG (CD162), LTBR, PAG/Cbp, NKp44, NKp30, NKp46, NKG2D, and/or NKG2C.

In a preferable embodiment, the transmembrane region of CAAR can be selected from transmembrane regions of proteins such as CD8 or CD28. Human CD8 protein is a heterodimer composed of two chains, αβ or γδ. In a preferred embodiment of the present disclosure, the transmembrane region is selected from the transmembrane region of CD8 (CD8a) or CD28. The CD8 hinge region (hinge) is a flexible region. Therefore, the CD8 or CD28 transmembrane region plus the hinge region can be used to connect the target recognition domain scFv of CAAR with the intracellular signal region.

In a preferable embodiment, the nucleic acid sequence of the transmembrane domain encodes the CD8 hinge and/or transmembrane domain.

The cytoplasmic domain or other intracellular signaling region (intracellular signaling domain) of the CAAR in the present disclosure is responsible for activating at least one normal effector function of the immune cell carrying the CAAR. "Effector function" refers to a specialized function of a cell. For example, the effector function of a T cell can be cytolytic activity or helper activity, including secretion of cytokines. Thus, the term "intracellular signaling region" refers to the portion of a protein that transduces effector function signals and directs the cell to perform specialized functions. Although the entire intracellular signaling region can be used, in many cases it is not necessary to use the entire domain. Regarding the use of truncated portions of intracellular signaling regions, such truncated portions can be used in place of the full domain as long as they can transduce effector function signals. Thus, "intracellular signaling region" is intended to include any truncated portion of an intracellular domain sufficient to transduce an effector function signal.

Examples of intracellular signaling transduction domains for use in CAARs of the present disclosure include, but are not limited to: the cytoplasmic portion of the T cell receptor (TCR) and co-receptors, and any derivatives or variants of these elements as well as those having the same functional capabilities. Examples of intracellular signaling regions comprise fragments or domains from one or more molecules or receptors, including but not limited to: CD3ζ, CD3γ, CD3δ, CD3ε, CD86, common FcRy, FcRβ (FcεR1b), CD79a, CD79b, Fc yRIIa, DAP10, DAP12, T cell receptor (TCR), CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, ligands that specifically bind to CD83, CDS, ICAM-1, GITR, BAFFR, HVEM (LIGHTR), SLAMF7, NKp80 (KLRF1), CD127, CD160, CD19, CD4 , CD8α, CD8β, IL2RP, IL2Ry, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b , ITGAX, CD11c, ITGB1, CD29, ITGB2, CD18, LFA-1, ITGB7, TNFR2, TRANCE/RANKL, DNAM1(CD226), SLAMF4(CD244, 2B4), CD84, CD96, CEACAM1, CRTAM, Ly9(CD229), CD160(BY55), PSGL1, CD100(SEMA4D), CD69, SLAMF6(NTB-A, Ly108), SLAM(SLAMF1, CD150, IPO-3), BLAME(SLAMF8), SELPLG(CD162), LTBR, LAT, GADS, SLP-76, PAG/Cbp, NKp44, NKp30, NKp46, NKG2D. Derivatives, variants or fragments thereof, any synthetic sequence of co-stimulatory molecules with the same functional capabilities, and any combination thereof are also included. Costimulatory molecules are cell surface molecules other than antigen receptors or their ligands that are required for lymphocytes to respond efficiently to antigens.

In an optional embodiment, the intracellular signaling region can be selected from the intracellular signaling regions of CD3ζ, FcεRIγ, CD27, CD28, 4-1BB, CD134, ICOS, GITR protein, and combinations thereof. The CD3 molecule is composed of five subunits, wherein the CD3ζ subunit (also known as CD3zeta, referred to as Z) contains three ITAM motifs. The motif is an important signal transduction region in the TCR-CD3 complex. FcεRIγ is mainly distributed on the surface of mast cells and eosinocytes, comprising an ITAM motif, which is similar to CD3ζ in structure, distribution and function. In addition, CD28, 4-1BB, and CD134 are co-stimulatory signal molecules. Upon binding with their respective ligands, the intracellular signaling regions of these molecules generate co-stimulatory effects, leading to sustained proliferation of immune effector cells (mainly T lymphocytes). This enhances the secretion of cytokines such as IL-2 and IFN-γ by immune effector cells and simultaneously improves the survival duration of CAAR immune effector cells in vivo. In a preferred embodiment, the intracellular signaling region of CAAR comprises CD3ζ signaling domain and 4-1BB co-stimulatory domain.

In the CAAR of the present disclosure, the truncated fragment can be operatively linked to the hinge region sequence and the transmembrane region sequence, and then operatively connected to the intracellular signal region.

In the examples of the present disclosure, preferable CAAR is provided by the inventors. In addition, it should be understood that on the basis of the optimized CAAR of the present disclosure, CAAR with some changes or modifications can also be included in the present disclosure. For example, the CAAR of the present disclosure can be selected from but not limited to the following specific sequential configurations: the truncated fragment or variant thereof-CD8-CD3ζ, the truncated fragment or variant thereof-CD8-CD137-CD3ζ, the truncated fragment or variant thereof-CD28a-CD28b-CD3ζ, or the truncated fragment or variant thereof-CD28a-CD28b-4-1BB-CD3ζ.

As an optional embodiment of the present disclosure, CAAR also comprises a leader sequence at the N-terminal of the extracellular antigen recognition domain, wherein the leader sequence may optionally be cleaved from the antigen recognition domain (e.g., scFv) during the cellular processing and localization of the CAAR to the cell membrane.

The present disclosure also comprises nucleic acids encoding the CAAR. The nucleic acid sequence of the present disclosure can be in the form of DNAs or RNAs. DNAs include cDNAs, genomic DNAs, or synthetic DNAs. DNAs can be single stranded DNAs or double stranded DNAs. DNAs can be coding or noncoding strand DNAs. Nucleic acid codon encoding the amino acid sequence of the CAAR protein of the present disclosure may be degenerate, that is, multiple degenerate nucleic acid sequences encoding the same amino acid sequence are all included in the scope of the present disclosure. The degenerate nucleic acid codons encoding corresponding amino acids are well known in the art.

The present disclosure also comprises variants of the above-mentioned polynucleotides, wherein the variants encode polypeptides, or fragments, analogues and derivatives of the polypeptides, with the same amino acid sequences as the present disclosure. The variants may be naturally occurring allelic variants or non-naturally occurring variants. These nucleotide variants include degenerate variants, substitution variants, deletion variants, and insertion variants. As known in the art, an allelic variant is an alternative form of a polynucleotide, which may be the substitution, deletion, or insertion of one or more nucleotides, but does not substantially alter the function of the polypeptide it encodes.

The present disclosure also provides genetically modified immune effector cells, wherein the cells are transduced with the nucleic acid of the present disclosure or transduced with the above-mentioned recombinant plasmid comprising the nucleic acid of the present disclosure, or the virus comprising the plasmid. The cells are cells or a cell population comprising the cells, preferably T cells or a cell population comprising T cells.

Conventional nucleic acid transduction methods in the art, comprising non-viral and viral transduction methods, can be used in the present disclosure. Non-viral based transduction methods comprise electroporation and transposon.

The present disclosure also provides an expression construct (vector) comprising the nucleic acid encoding the CAAR protein expressed on the surface of immune effector cells, including viral vectors or non-viral vectors.

The non-viral vector system such as the Sleeping Beauty system (Sleeping Beauty system) or PiggyBac transposon have a higher efficiency of transduction than ordinary electroporation. The combined application of nucleofector transfection instrument and Sleeping Beauty system has been reported [Davies JK., et al. Cancer Res, 2010, 70(10): OF1-10.]. This method not only has a high transduction efficiency but also allows for targeted integration of the gene of interest. In addition, mRNA transfection is also applicable.

A variety of vectors for viral packaging can be used in the present disclosure, including lentiviral vectors, adenoviral vectors, adeno-associated viral vectors, as well as viral vectors formed on the basis of these viral vectors after further modification. In a specific embodiment of the present disclosure, the backbone vector used in the present disclosure is a lentiviral plasmid vector. In this vector, the expression of CAAR molecules is driven by an EF1 promoter. The CAAR lentiviral vector plasmid can be co-transfected into cells (such as 293T cells) for producing virus in the presence of auxiliary packaging plasmids and can be packaged as lentiviruses carrying CAAR molecules. In addition, it should be understood that although specific lentiviral vectors are preferred in the present disclosure, other types of vectors are also applicable, as long as active CAAR and modified immune effector cells thereby can be finally obtained.

The present disclosure also comprises viruses packaged from viral vectors. The viruses may be lentiviruses, adenoviruses, adeno-associated viruses, and viruses further modified on the basis of these viruses are also included. The viruses of the present disclosure comprise packaged infectious viruses, as well as viruses to be packaged that comprises the components necessary for packaging as infectious viruses. It should be understood that although lentivirus is preferably used in the present disclosure, other viruses known in the art that can be used to transfer foreign genes into immune effector cells and their corresponding plasmid vectors can also be used in the present disclosure.

In one embodiment of the present disclosure, the transduction method for achieving CAAR gene-modified immune effector cells is a transduction method based on viruses such as lentiviruses. This method has the advantages of high transduction efficiency, stable expression of exogenous genes and can shorten the time for culturing immune effector cells in vitro to reach clinical-grade quantities. On the surface of the transgenic immune effector cells, the transduced nucleic acid is expressed on the surface through transcription and translation.

The immune cells of the present disclosure can also carry coding sequences of exogenous cytokines; the cytokines include but are not limited to: IL-12, IL-15 or IL-21 and so on. These cytokines have immunomodulatory activity and can enhance the function of effector T cells and activated NK cells. Therefore, those skilled in the art can understand that the use of these cytokines helps the immune cells to function better.

The immune cells of the present disclosure can also express a safety switch; preferably, the safety switch comprises: iCaspase-9, Truncated EGFR or RQR8. As mentioned earlier, current CA therapy is also challenged by the complexity of its production and adverse events related to cellular activity, such as cytokine storm (CRS). Therefore, drugs or therapies that can effectively regulate CAAR-T, such as setting a safety switch, are more preferred.

### Pharmaceutical composition

The genetically modified immune effector cells of the present disclosure can be used for preparing compositions, especially pharmaceutical compositions. Besides the effective amount of the immune effector cells, the composition may also comprise a pharmaceutically acceptable carrier. The term "pharmaceutically acceptable" means that the molecular entities and compositions do not produce adverse, allergic or other adverse reactions when properly administered to animals or humans.

The truncated fragment of the present disclosure or the fused protein comprising it can be used as an antigen, and can also be used in the preparation of compositions, such as pharmaceutical compositions. Besides the effective amount of the truncated fragment, the composition may also comprise a pharmaceutically acceptable carrier.

Specific examples of substances that may be used as pharmaceutically acceptable carriers or components thereof are polyhydric alcohols such as propylene glycol, glycerin, sorbitol, mannitol and polyethylene glycol; alginic acid; emulsifiers such as Tween^{®}; wetting agents such as sodium lauryl sulfate; coloring agents; flavoring agents; excipients, stabilizers; antioxidants; preservatives; pyrogen-free water; isotonic saline solution; sugars such as lactose, glucose and sucrose; starches, such as cornstarch and potato starch; cellulose and its derivatives, such as sodium carboxymethylcellulose, ethylcellulose, and methylcellulose; powdered tragacanth; malt; gelatin; talc; solid lubricants like stearic acid and magnesium stearate; calcium sulfate; vegetable oils such as peanut oil, cottonseed oil, sesame oil, olive oil, corn oil and cocoa butter; phosphate buffer, etc.

The compositions of the present disclosure can be formulated into various dosage forms as needed and administered in dosages beneficial to the patient, as determined by a physician based on factors such as the type of patient, age, body weight, general condition of the disease, and administration method. Administration method can be for example injection or other therapeutic methods.

When considering "immunologically effective amount", "effective amount of anti-autoantibody", "effective amount for inhibiting autoimmune diseases" or "therapeutic amount", the precise amount of the composition of the disclosure to be administered can be determined by the physician taken into account of individual differences such as age, weight and the condition of patients (subjects). Generally, the pharmaceutical composition comprising the immune effector cells can be administered at a dose of 10³-10¹⁰ (such as 10⁴, 10⁵, 10⁶, 10⁷ or 10⁸) cells/kg body weight, in some embodiments such as 10⁵ -10⁸ cells/kg body weight. The composition may also be administered in multiples of these doses. Cells can be administered by using infusion generally known in immunotherapy. The optimal dosage and treatment regimen for a particular patient can be readily determined by one skilled in the medical arts by monitoring the patient for indications of disease and adjusting treatment accordingly.

The compositions of the present disclosure may also be provided in suitable kits for the convenience of clinicians. Preferably, the kit may also comprise an instruction manual explaining the method of using the composition of the present disclosure.

### Clinical Diagnosis/Type

The truncated fragment of GPIbα described in the present disclosure can be used to prepare a diagnostic antigen, wherein the antigen can be used to clarify the autoantibody binding epitope and relative titer of patients with immune thrombocytopenia (ITP), so as to identify patients with different clinical types.

As an alternative embodiment, reagents that can be used to detect autoantibodies can be designed based on the principle of indirect ELISA. For example, the truncated fragment of the present disclosure can be connected to a solid phase carrier, and the autoantibody in the sample to be tested is combined with it to form a solid phase antigen-tested autoantibody complex. Then the enzyme-labeled secondary antibody and the solid phase antigen-tested autoantibody complex are combined to form a solid phase antigen-tested autoantibody-enzyme-labeled secondary antibody complex. The absorbance after adding the substrate is measured to determine the level of the tested autoantibody.

The truncated fragment can be used as an antigenic protein, for example, can be immobilized on a solid-phase carrier at a concentration of 10nM-100µM. The concentrations used for different antigenic proteins can be the same or different. Preferably, the truncated fragment is covalently immobilized on the surface of a solid-phase carrier. A specific concentration of human anti-tag peptide is used as a calibrator for quantitative detection. The signal intensity is measured using colorimetric, fluorescence, or chemiluminescence methods for detecting autoantibodies. The signal detection methods used in the detection comprise but are not limited to visible light colorimetry, fluorescence luminescence, chemiluminescence and so on.

Based on the new discovery of the present disclosure, a kit for diagnosing, prognosing or typing ITP is also provided.

As an embodiment, the kit comprises a solid phase carrier coated with the antigen protein. The detection kit can be designed to be suitable for visible light colorimetry, fluorescence luminescence or chemiluminescence detection. The solid phase carrier may be, but not limited to: magnetic particles, microspheres, plastic beads, liquid phase chips, microwell plates, affinity membranes, glass slides, test papers and so on. For example, the antigenic protein is covalently immobilized on the solid phase carrier. Preferably, visible light colorimetry, fluorescence luminescence and/or chemiluminescence detection is achieved by coating the antigen on a microwell plate, such as a 96-well plate.

The immobilization method of the antigenic protein comprises direct coating method and indirect coating method. As a specific embodiment, the antigen is mixed with an appropriate premix solution and incubated to activate the antigen, and then added to a solid phase carrier, incubated and blocked. In addition, the indirect coating method indirectly immobilizes the antigenic protein on the solid phase carrier, for example, through a specific reaction between biotin and streptavidin.

According to the present disclosure, the kit also comprises: diluent (such as sample and antibody diluent), secondary antibody, washing solution, antibody calibrator tagged with humanized peptide, quality control of the antibody tagged with humanized peptide, substrate, stop solution, luminescence solution, etc. Preferably, the second antibody has a detectable label; the detectable label is, for example, horseradish peroxidase.

The disclosure if further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press) or followed the manufacturer's recommendation.

### Example 1. Design of different truncated fragments of GpIbα gene

To determine the binding sites between GpIbα and endogenous autoimmune antibodies, it is considered to study the binding sites of GpIbα and autoantibodies to elucidate the antigen-antibody binding sites. Therefore, based on the sequence of bases in the extracellular domain of human GpIbα, multiple specific primer sequences were designed. Using PCR technology, truncated gene fragments targeting different antigenic epitopes of GpIbα were obtained. Five different truncated fragments were screened, with antigenic epitopes on GpIbα as follows: 1H-290R, 265P-414P, 265P-509F, 1H-509F, and 1H-414P (numbered 1# to 5#). The specific nucleotide/amino acid sequences are as follows:
The truncated fragment 1#: 1H-290R (GPIbα-1#) has a nucleotide sequence as shown in SEQ ID NO: 1 and an amino acid sequence as shown in SEQ ID NO: 2; The amino acid sequence with G233K mutation is as shown in SEQ ID NO: 17, with a corresponding coding sequence as shown in SEQ ID NO: 16.
The truncated fragment 2#: 265P-414P(GPIbα-2#) has a nucleotide sequence as shown in SEQ ID NO: 3 and an amino acid sequence as shown in SEQ ID NO: 4.
The truncated fragment 3#: 265P-509F(GPIbα-3#) has a nucleotide sequence as shown in SEQ ID NO: 5 and an amino acid sequence as shown in SEQ ID NO: 6.
The truncated fragment 4#: 1H-509F(GPIbα-4#) has a nucleotide sequence as shown in SEQ ID NO: 7 and an amino acid sequence as shown in SEQ ID NO: 8; The amino acid sequence with G233K mutation is as shown in SEQ ID NO: 19, with a corresponding coding sequence as shown in SEQ ID NO: 18.
The truncated fragment 5#: 1H-414P(GPIbα-5#) has a nucleotide sequence as shown in SEQ ID NO: 9 and an amino acid sequence as shown in SEQ ID NO: 10; The amino acid sequence with G233K mutation is as shown in SEQ ID NO: 21, with a corresponding coding sequence as shown in SEQ ID NO: 20.

### Example 2. Construction and screening of GpIbα-CAAR lentiviral vectors of different truncated fragments

### (1) Synthesis of the second-generation CAR structural gene

The nucleotide fragments of the CAR structural gene encoding the CD8 hinge and transmembrane region and the intracellular segment including the 4-1BB co-stimulatory domain and the CD3ζ signaling domain were obtained by PCR. The second-generation CAR structural gene was fused with previously screened truncated gene fragments targeting different GpIbα epitopes by overlapping PCR to obtain the GpIbα-CAAR sequence. Currently, five different truncated GpIbα-CAAR fragments (numbered 1# to 5#) have been successfully constructed, as detailed below:
CAAR1#: An amino acid sequence of CAAR corresponding to 1H-290R is shown in SEQ ID NO: 11; wherein residues 1 to 290 is the antibody recognition sequence of GpIbα, residue 233 of SEQ ID NO: 11 has a G233K mutation, residues 291 to 335 is CD8 hinge domain, residues 336 to 359 is CD8 transmembrane region, residues 360 to 401 is 4-1BB domain, residues 402 to 513 is CD3ζ signaling domain.
CAAR2#: An amino acid sequence of CAAR corresponding to 265P-414P is shown in SEQ ID NO: 12; wherein residues 1 to 150 is the antibody recognition sequence (265P-414P sequence), residues 151 to 373 comprises CD8 hinge domain, CD8 transmembrane region, 4-1BB domain and CD3ζ signaling domain.
CAAR3#: An amino acid sequence of CAAR corresponding to 265P-509F is shown in SEQ ID NO: 13; wherein residues 1 to 245 is the antibody recognition sequence (265P-509F sequence), residues 246 to 373 comprises CD8 hinge domain, CD8 transmembrane region, 4-1BB domain and CD3ζ signaling domain.
CAAR4#: An amino acid sequence of CAAR corresponding to 1H-509F is shown in SEQ ID NO: 14; wherein residues 1 to 509 is the antibody recognition sequence (1H-509F sequence), residue 233 of SEQ ID NO: 14 has a G233K mutation, residues 510 to 732 comprises CD8 hinge domain, CD8 transmembrane region, 4-1BB domain and CD3ζ signaling domain.
CAAR5#: An amino acid sequence of CAAR corresponding to 1H-414P is shown in SEQ ID NO: 15; wherein residues 1 to 414 is the antibody recognition sequence (1H-414P sequence), residue 233 of SEQ ID NO: 15 has a G233K mutation, residues 415 to 637 comprises CD8 hinge domain, CD8 transmembrane region, 4-1BB domain and CD3ζ signaling domain.

### (2) Construction of Pre-Lenti-EF1-GpIbα-CAAR lentiviral vector

The GpIbα-CAAR gene sequence was analyzed for selecting restriction sites that match the multiple cloning site (MCS) of the Pre-Lenti-EF1-MCS lentiviral vector. After restriction digestion, ligation, transformation, clones were picked for bacterial amplification, with plasmids extracted and sequenced. Homologous recombination was used to construct the Pre-Lenti-EF1-GpIbα-CAAR lentiviral vector.

### (3) Packaging and titer detection of GpIbα-CAAR lentivirus

293T cells were transfected according to a ratio of GpIbα-CAAR:PsPAX2:PMD2G=3:2:1, with the virus supernatant collected at 48 hours and 72 hours. The virus was concentrated with PEG8000. An appropriate amount of virus concentrate was used for determining virus titer by Takara lentivirus kit, with others frozen at -80°C for later use.

### Example 3. Preparation of GpIbα-CAAR-T cells

Human peripheral blood mononuclear cells (PBMCs) were obtained using Ficoll density gradient centrifugation. T cells were obtained from PBMCs by immunomagnetic bead separation. The isolated T cells were added to a T75 culture flask coated with 5 µg/mL of CD3 and CD28 antibodies, and 1000 U/mL IL-2 and 100 U/mL IFN-y were added to activate the T cells. After two days, a GpIbα-CAAR viral supernatant comprising different truncated fragments was added to the activated T cells for virus packaging. After culturing for 48h to 72h, the viral supernatant was removed. The infection efficiency of the virus was detected by flow cytometry. GpIbα-CAAR-T cells were obtained. Additional part of activated T cells was cultured and expanded normally, without transfection of CAAR, and used as negative control T cells.

### Example 4. Construction of GpIbα-CAAR-T cells with mutations in the von Willebrand Factor (vWF) binding region

### (1) Construction of the GpIbα-CAAR plasmid with a point mutation in the vWF binding region

In order to reduce the possible off-target effects after GpIbα-CAAR-T cell infusion in vivo, based on the successfully designed GPIbα-CAAR plasmid, the inventors carried out a point mutation on the binding region of GpIbα to vWF. The glycine (G) of residue 233 in GpIbα epitope for binding to the vWF-A1 functional domain was mutated to lysine (K). A GpIbα-CAAR-G233K mutant plasmid was constructed.

### (2) Detection of the binding ability of GpIbα-CAAR-G233K mutant to WF

The constructed GpIbα-CAAR-G233K mutant plasmid was used to infect Jurkat cells with lentivirus to establish a stable transfected cell line. Under the action of ristocetin, Jurkat cells stably expressing CAAR1, CAAR4 and CAAR5 mutants were interacted with human vWF. Cell aggregation was measured (with the results showed by the light transmission).

The results are shown in Figure 1. In the presence of vWF, the aggregation of Jurkat cells expressing CAAR1, CAAR4 and CAAR5 mutants was significantly lower than that of Jurkat cells without point mutations (decreased light transmission), indicating that Jurkat cells stably expressing CAAR1, CAAR4 and CAAR5 mutants showed a significantly decreased ability of binding with human vWF.

Therefore, the GpIbα-G233K mutant could alleviate off-target effects after CAAR-T-cell infusion in vivo. The present disclosure successfully prepared a series of CAAR-T cells with mutations in the binding region of vWF.

### Example 5. Anti-human GpIbα monoclonal hybridoma cell preparation

### (1) Immunization of BALB/c mice with human GpIbα protein

Human GpIbα protein was injected into the footpad of BALB/c mice for immunization. Complete Freund's adjuvant mixed with the GpIbα antigen for emulsion was used for the first injection of animal immunization. For the second or third injection, incomplete adjuvant or no adjuvant was used. The prepared antigen emulsion was injected into one footpad of mice with a syringe. Generally, each mouse was injected with 50-100 µg of protein antigen for each injection and the volume of each injection of antigen does not exceed 300 µL. Splenocytes were collected for fusion on the 3rd day after the final booster immunization.

### (2) Preparation of hybridoma cells by cell fusion

After three rounds of immunization, mouse spleen B cells and myeloma cells SP2/0 were fused with PEG-1500 and hybridoma cells were screened using HAT medium. Successfully fused cells could grow in HAT medium, while unsuccessfully fused B cells will die within 5 to 7 days.

### (3) Screening monoclonal hybridoma cells

Hybridoma cells survived in the HAT medium were counted, with the concentration adjusted to about 200 cells/mL. 4 mL of semi-solid medium was spread in a 6-well plate, and then about 500 µL of hybridoma cell suspension was added to each well. After 7 days of culture, single clones were picked out and transferred to a new 6-well plate for continuous culture and gradual cell expansion.

### (4) Selection of anti-human GpIbα-positive monoclonal hybridoma cells

Monoclonal hybridomas were isolated. The combination of hybridoma culture supernatant and GpIbα protein was detected by Elisa. The hybridoma cells with positive Elisa results were selected. The hybridoma cells produce anti-human GpIbα antibodies (anti-human platelet antibodies).

### (5) Verification of GpIbα-CAAR-T Cells binding to anti-GpIbα positive hybridoma cells

The previously designed GpIbα-CAAR-T cells with different truncated fragments were co-incubated with the obtained anti-GpIbα positive hybridoma cells. Flow cytometry was used to detect the binding ability of GpIbα-CAAR-T cells to the antibodies in the supernatant of the monoclonal hybridoma cells with different antigen epitopes, with their binding abilities evaluated.

As shown in Figure 2, the CAAR-T cells designed by the inventors can selectively bind to anti-GpIbα positive hybridoma cells (Gvb1-Gvb4) with different antigen epitopes, and their binding abilities vary. Among them, CAAR2-T cells and CAAR3-T cells have the ability to bind to Gvb4 hybridoma cells, CAAR4-T cells and CAAR5-T cells can strongly bind to the four hybridoma cells Gvb1-Gvb4, and the G233K point mutation will not affect the specific binding of CAAR-T cells and anti-GpIbα-positive hybridoma cells. Therefore, different CAAR-T cells containing G233K point mutations all retain the ability to recognize target B cells, wherein CAAR1-T cells have the strongest recognition ability.

### Example 6. In-vitro killing efficiency of GpIbα-CAAR-T cells to anti-GpIbα-positive hybridoma cells

### (1) Competitive cytotoxicity assay

Anti-GPIba positive hybridoma cells were labeled with CellTracker^{™} Deep Red Dye and negative control hybridoma cells were stained with CellTracker^{™} Red CMTPX Dye. The two were mixed according to a certain ratio, with GpIbα-CAAR-T cells added. After the three were co-cultured for 16 hours, flow cytometry was used to detect the ratio of the two hybridoma cells with different fluorescent labels, so as to reflect the killing efficiency of CAAR-T cells on the target cells.

As shown in Figure 3, CAAR1-T cells to CAAR5-T cells (corresponding to 1#CAAR-T to 5#CAAR-T, respectively) can selectively kill positive hybridoma cells with different antigen epitopes, resulting in varying degrees of reduction in the proportion of positive hybridoma cells. Among them, CAAR2-T cells and CAAR3-T cells exhibit cytotoxicity against Gvb4 hybridoma cells, CAAR4-T and CAAR5-T cells show strong cytotoxicity against Gvb1 and Gvb2 hybridoma cells and CAAR1-T cells demonstrate strong competitive cytotoxicity against all four types of positive hybridoma cells (Gvb1-Gvb4), with significant statistical differences compared to the control group. It is indicated that CAAR-T cells with different antigen epitopes can specifically recognize and kill anti-GpIbα positive target cells, wherein CAAR1-T cells showing a strong competitive cytotoxicity against all target cells.

### (2) LDH release assay

To assess the specific cytotoxicity of GpIbα-CAAR-T cells, the inventors measured the LDH release after co-culturing CAAR-T cells including different antigen epitopes with anti-GpIbα hybridoma cells for 16 hours.

As shown in Figure 4, the constructed CAAR-T cells can effectively recognize anti-GpIbα positive hybridoma cells targeting different antigen epitopes and exhibit a certain degree of cytotoxicity. Among them, CAAR1-T cells demonstrate cytotoxicity against all four types of positive hybridoma cells (Gvb1-Gvb4) at different effect-to-target ratios, with a stronger effect of lysing cells compared to CAAR4-T and CAAR5-T cells.

### 3) Cytokine release assay

GpIbα-CAAR-T cells were mixed with anti-GpIbα positive hybridoma cells at a certain ratio and co-cultured for 24 hours. ELISA was used to detect the levels of IL-2 and IFN-y in the supernatant samples, reflecting the cytotoxic capacity of CAAR-T cells against target cells.

As shown in Figure 5, GpIbα-CAAR-T cells can effectively recognize and kill anti-GpIbα positive target cells, exhibiting a certain degree of cytotoxicity. Wherein, CAAR1-T cells demonstrate cytotoxicity against all four types of positive hybridoma cells (Gvb1-Gvb4), with significantly higher levels of released IL-2 and IFN-y compared to CAAR4-T and CAAR5-T cells. It is suggested that CAAR1-T cells have stronger cytotoxicity against target cells, enabling target elimination of reactive B cells in vivo and reducing the production of anti-platelet antibodies or free antibodies.

### Example 7. Construction and in-vivo verification of humanized ITP mouse model

Using humanized NSG mice, an ITP-hybridoma animal model was constructed. Specifically, six-week-old NSG (NOD-Prkdcscid il-2rgtm1/Bcgen) immunodeficient mice were irradiated to ablate their bone marrow. Human umbilical cord blood or fetal hematopoietic stem cells (HSCs) were transplanted into these irradiated mice, ensuring continuous production of various human hematopoietic or immune cells, thereby completing the humanization of the mice. Subsequently, humanized mice were intraperitoneally injected with 1×10⁶ anti-GpIbα positive hybridoma cells. Survival status of the mice was monitored daily. Platelet counts in mice, PA Abs levels in peripheral blood, the number of megakaryocytes in bone marrow, the changes in coagulation function and other indicators were regularly detected. These indicators were used to judge whether the ITP animal model of mice is established.

A certain number of GpIbα-CAAR-T cells were selectively injected into the tail veins of successfully constructed ITP-hybridoma mice. The titer of anti-GPIbα autoantibodies in mice, platelet counts, coagulation changes and other indicators were evaluated for comprehensive assessment of the killing efficiency of CAAR-T cells with different truncated fragments on hybridoma cells in vivo, thereby clarifying that CAAR-T cells can improve the induced ITP disease model in mice. As shown in Figure 6, at the 14th day, the control group (NTD) mice showed significant Gvbl-GFP expansion and metastasis, while the CAAR-T cell-treated group exhibited significantly lower Gvb1-GFP cell expansion and metastasis. At the 21st day, the control group (NTD) mice displayed a significant degree of Gvb1-GFP expansion and metastasis, whereas the CAAR-T cell-treated group had significantly lower Gvbl-GFP cell expansion and metastasis, demonstrating a clear cytotoxic effect of CAAR-T cells on Gvb1 cells.

### Example 8. Selection and application of GpIbα-CAAR-T cells

### (1) Collection, Isolation, and Activation of T Cells

A sufficient number of T cells were obtained from volunteer subjects using Ficoll separation or apheresis. The T cells were washed and activated in a sterile environment, and cultured with essential cytokines necessary for survival and proliferation. CD3 antibodies and CD28 antibodies are the most commonly used for T cell activation in vitro.

### (2) Construction and selection of different truncated GpIbα-CAAR-T cells

GpIbα-CAAR gene fragments comprising different truncated fragments were transduced into activated T cells. Common methods comprise lentiviral vectors, retroviral vectors, transposons, mRNA electroporation, and so on. Based on the types of autoantibodies identified in ITP patients, the inventors selectively used GpIbα-CAAR-T cell therapies containing different truncated fragments.

### (3) In Vitro proliferation and culture of GpIbα-CAAR-T Cells

Genetically modified CAAR-T cells were stimulated and cultured in vitro to reach a sufficient number. The quality of the cells were controlled before intravenous infusion into the body. Typically, tens to hundreds of billions of CAAR-T cells are required.

Figure 7 shows that when activated T cells were infected with lentiviral vectors containing different truncated GpIbα-CAAR gene fragments and cultured for a period of time, flow cytometry results indicated that lentiviruses with different truncated fragments could infect a certain proportion of human T cells, thus constructing GpIbα-CAAR-T cells containing different truncated fragments.

Above-described examples only show several embodiments of the present disclosure, which are described specifically and in detail. However, it should not be understood as a limiting patent scope of the present disclosure. It should be noted that those skilled in the art can make some adjustments and improvements without departing from the concept of the present disclosure and all these forms are within the scope of protection of the present disclosure. Therefore, the scope of patent protection in the present disclosure should be determined by the appended claims. Simultaneously, each reference provided herein is incorporated by reference to the same extent as if each reference was individually incorporated by reference.

## Claims

1. A use of a platelet surface antigen GpIbα, a truncated fragment, a variant, or an encoding polynucleotide thereof, for: (a) preparing a competitive antigen or a construct or composition comprising the antigen, the competitive antigen can bind to anti-platelet antibody, (b) preparing a construct or composition for targeting and eliminating reactive B lymphocytes, (c) preparing a construct or composition for increasing platelets, (d) preparing a construct or composition for alleviating or treating immune thrombocytopenic purpura.

2. A truncated fragment of platelet surface antigen GpIbα, a variant, or an encoding polynucleotide thereof, wherein the truncated fragment or the variant has following characteristics: it is derived from an extracellular region of the GpIbα protein and it has binding sites for in vivo platelet autoantibody; more preferably, the truncated fragment or the variant comprises one or more proteins with following sequences: SEQ ID NO: 2, SEQ ID NO: 4, SEQ ID NO: 6, SEQ ID NO: 8, SEQ ID NO: 10, SEQ ID NO: 17, SEQ ID NO: 19 or SEQ ID NO: 21.

3. A chimeric autoantibody receptor, comprising: the truncated fragment of platelet surface antigen GpIbα or the variant according to claim 2 as an extracellular binding region, a transmembrane domain and an intracellular signaling domain; preferably, the intracellular signaling domain comprises: co-stimulatory molecules and/or signaling transduction domains.

4. The chimeric autoantibody receptor according to claim 3, wherein, the transmembrane domain comprises a CD8 or CD28 hinge region and transmembrane region; preferably, the transmembrane domain comprises a CD8 hinge region and transmembrane region; more preferably, the hinge region has an amino acid sequence of residues 291 to 335 as shown in SEQ ID NO: 11, the transmembrane region has an amino acid sequence of residues 336 to 359 as shown in SEQ ID NO: 11; or
the intracellular signaling domain comprises an intracellular signaling region selected from 4-1BB, CD3ζ, FcεRIγ, CD27, CD28, CD134, ICOS or GITR; preferably, the intracellular signaling region comprises 4-1BB and CD3ζ; more preferably, the 4-1BB has an amino acid sequence of residues 360 to 401 as shown in SEQ ID NO:11, the CD3ζ has an amino acid sequence of residues 402 to 513 as shown in SEQ ID NO:11.

5. The chimeric autoantibody receptor according to claim 3 or 4, wherein, the chimeric autoantibody receptor comprises following sequentially connected structures: the truncated fragment of platelet surface antigen GpIbα or the variant according to claim 2 or 3, a CD8 or CD28 hinge region and transmembrane region, 4-1BB and CD3ζ; preferably, the chimeric autoantibody receptor has any one of amino acid sequences as shown in SEQ ID NO: 11 to SEQ ID NO: 15.

6. An expression construct, comprising:
(i) a polynucleotide encoding the chimeric autoantibody receptor according to any one of claims 3 to 5; or
(ii) a polynucleotide encoding the truncated fragment of platelet surface antigen GpIbα or the variant according to claim 2.

7. An immune effector cell, wherein it is introduced with: the polynucleotide of the chimeric autoantibody receptor according to any one of claims 3 to 5 or the expression construct of (i) in claim 6; or expressing the chimeric autoantibody receptor according to any one of claims 3 to 5;
preferably, the immune effector cell comprises cells selected from the following group or a combination thereof: T cell, natural killer cell, NKT cell or cytokine-induced killer cell; more preferably, the immune effector cell is T cell; more preferably, the T cell comprises: helper T cell, cytotoxic T cell, memory T cell, regulatory T cell, γδ T cell, T memory stem cell or T cell derived from pluripotent stem cell.

8. A use of the immune effector cell according to claim 7, for: (a) preparing a composition for neutralizing anti-platelet antibody or free antibody, (b) preparing a composition for targeting and eliminating reactive B lymphocytes, (c) preparing a composition for increasing platelets, (d) preparing a composition for alleviating or treating immune thrombocytopenic purpura.

9. A pharmaceutical composition or drug kit, comprising the immune effector cell according to claim 7, the pharmaceutical composition or drug kit is used for: (a) neutralizing anti-platelet antibody or free antibody, (b) targeting and eliminating reactive B lymphocytes, (c) increasing platelets, (d) alleviating or treating immune thrombocytopenic purpura.

10. A method for (a) neutralizing anti-platelet antibody or free antibody, (b) targeting and eliminating reactive B lymphocytes, (c) increasing platelets, (d) alleviating or treating immune thrombocytopenic purpura, wherein the method comprises: administering an effective amount of the immune effector cell according to claim 7 or the pharmaceutical composition according to claim 10 to a subject.
